# EUROPEAN PATENT SPECIFICATION

(11) **EP 2 162 129 B1**
(45) Date of publication and mention of the grant of the patent: **06.03.2019**
(21) Application number: 08756347.4
(22) Date of filing: 28.05.2008
(51) Int. Cl.: A61K 31/4045, A61P 19/08, A61P 19/10, A61P 35/00

(54) **USE OF HDAC INHIBITORS FOR THE TREATMENT OF BONE DESTRUCTION**
VERWENDUNG VON HDAC HEMMERN ZUR BEHANDLUNG VON KNOCHENZERSTÖRUNG
UTILISATION D'INHIBITEURS DE HDAC POUR LE TRAITEMENT DE LA DESTRUCTION OSSEUSE

(30) Priority: 30.05.2007 US 940735 P
(43) Date of publication of application: 17.03.2010
(73) Proprietor: Novartis AG, 4056 Basel (CH)
(72) Inventor: ATADJA, Peter, Wisdom, Acton, Massachusetts 01720 (US); GROWNEY, Joseph, Daniel, Reading, Massachusetts 01867 (US); SHAO, Wenlin, Dedham, Massachusetts 02026 (US)
(74) Representative: Roth, Peter Richard
(86) International application number: PCT/US2008/064944
(87) International publication number: WO 2008/150782

(56) References cited:
- WO-A-02/22577
- FENG RENTIAN ET AL: "A novel, mercaptoketone-based HDAC inhibitor, KD5170 exerts marked inhibition of osteoclast formation and anti-myeloma activity in vitro." BLOOD, vol. 108, no. 11, Part 1, November 2006 (2006-11), pages 991A-992A, XP002490854 & 48TH ANNUAL MEETING OF THE AMERICAN-SOCIETY-OF-HEMATOLOGY; ORLANDO, FL, USA; DECEMBER 09 -12, 2006 ISSN: 0006-4971
- FENG RENTIAN ET AL: "Combination of the proteasome inhibitor bortezomib and a histone deacetylase inhibitor PXD101 results in synergistic inhibition of osteoclastogenesis and significantly stronger inhibition of multiple myeloma growth in vitro and in vivo." BLOOD, vol. 108, no. 11, Part 1, November 2006 (2006-11), pages 153A-154A, XP002490855 & 48TH ANNUAL MEETING OF THE AMERICAN-SOCIETY-OF-HEMATOLOGY; ORLANDO, FL, USA; DECEMBER 09 -12, 2006 ISSN: 0006-4971
- CHUNG Y -L ET AL: "A therapeutic strategy uses histone deacetylase inhibitors to modulate the expression of genes involved in the pathogenesis of rheumatoid arthritis" MOLECULAR THERAPY 200311 US, vol. 8, no. 5, November 2003 (2003-11), pages 707-717, XP002490857 ISSN: 1525-0016
- NAKAMURA TAKAHIRO ET AL: "Inhibition of histone deacetylase suppresses osteoclastogenesis and bone destruction by inducing IFN-beta production" JOURNAL OF IMMUNOLOGY, vol. 175, no. 9, November 2005 (2005-11), pages 5809-5816, XP002490858 ISSN: 0022-1767
- SCHROEDER TANIA M ET AL: "Histone deacetylase inhibitors promote osteoblast maturation" JOURNAL OF BONE AND MINERAL RESEARCH, vol. 20, no. 12, December 2005 (2005-12), pages 2254-2263, XP002490859 ISSN: 0884-0431
- LEE HYUN WOO ET AL: "Histone deacetylase 1-mediated histone modification regulates osteoblast differentiation" MOLECULAR ENDOCRINOLOGY, vol. 20, no. 10, October 2006 (2006-10), pages 2432-2443, XP002490860 ISSN: 0888-8809
- YI TACGHEE ET AL: "Trichostatin A-mediated upregulation of p21(WAF1) contributes to osteoclast apoptosis" EXPERIMENTAL & MOLECULAR MEDICINE, vol. 39, no. 2, April 2007 (2007-04), pages 213-221, XP002490861 ISSN: 1226-3613
- REVILL P ET AL: "Panobinostat: Histone deacetylase (HDAC) inhibitor, apoptosis inducer, oncolytic" DRUGS OF THE FUTURE, BARCELONA, ES, vol. 32, no. 4, 1 January 2007 (2007-01-01), pages 315-322, XP002457526 ISSN: 0377-8282
- GROWNEY JOSEPH D ET AL: "Efficacy of panobinostat (LBH589) in multiple myeloma cell lines and in vivo mouse model: Tumor-specific cytotoxicity and protection of bone integrity in multiple myeloma" BLOOD, vol. 110, no. 11, Part 1, November 2007 (2007-11), page 452A, XP002490862 & 49TH ANNUAL MEETING OF THE AMERICAN-SOCIETY-OF-HEMATOLOGY; ATLANTA, GA, USA; DECEMBER 08 -11, 2007 ISSN: 0006-4971

## Description

### Field of the Invention

The invention relates to the use of an histone deacetylase (HDAC) inhibitor, which is *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, for the manufacture of pharmaceutical compositions for the treatment of bone destruction associated with cancer. The present invention also relates to the HDAC inhibitor *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, for use in the treatment of bone destruction associated with cancer.

### Background of the Invention

The normal bone turnover is regulated by the balance between the osteolytic activity of osteoclasts and the bone forming activity of osteoblasts. Bone integrity may be compromised in patients suffering from cancer, inflammatory diseases and osteoporosis. Therefore, there is a need to develop novel treatment methods using HDAC inhibitors.

Feng et al (Blood, 2006 108: Abstract 3477) discloses the effect of the novel mercaptoketone-based HDAC inhibitor, KD5170, on the formation of osteoclasts from multiple myeloma (MM) patients and also discloses an antiproliferative activity on the MM cell line U266.

Feng et al (Blood, 2006 108: Abstract 507) discloses the effect of a combination of bortezomib and the HDAC inhibitor PDX101 on *in vitro* multiple myeloma cell lines and in an *in vivo* xenograft tumour model.

### Summary of the Invention

Reversible acetylation of histones is a major regulator of gene expression that acts by altering accessibility of transcription factors to DNA. In normal cells, histone deacetylase (HDAC) and histone acetyltransferase together control the level of acetylation of histones to maintain a balance. Inhibition of HDAC results in the accumulation of hyperacetylated histones, which results in a variety of cellular responses.

Surprisingly, it was now found that HDAC inhibitors, especially the compound of formula (III), as defined herein, treat bone destruction associated with cancer. More specifically the cancer is multiple myeloma, breast cancer or prostate cancer. Hence, the invention relates to the use of the HDAC inhibitor *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, for the preparation of a medicament for the treatment of bone destruction associated with a cancer which is multiple myeloma, breast cancer or prostate cancer. The invention also relates to the HDAC inhibitor, *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, for use in the treatment of bone destruction associated with a cancer which is multiple myeloma, breast cancer or prostate cancer.

Thus the invention provides the use of the HDAC inhibitor *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, for the preparation of a medicament:
- for the treatment of bone destruction caused by a proliferative cancer wherein the disease is selected from multiple myeloma, breast cancer or prostate cancer;
- for the prevention of bone destruction caused by a proliferative cancer wherein the disease is selected from multiple myeloma, breast cancer or prostate cancer; and
- for the treatment and prevention of bone destruction caused by a proliferative cancer wherein the disease is selected from multiple myeloma, breast cancer or prostate cancer,

The present invention also provides the HDAC inhibitor *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof,
- for use in the treatment of bone destruction caused by a proliferative cancer wherein the disease is selected from multiple myeloma, breast cancer or prostate cancer;
- for use in the prevention of bone destruction caused by a proliferative cancer wherein the disease is selected from multiple myeloma, breast cancer or prostate cancer; and
- for use in the treatment and prevention of bone destruction caused by a proliferative cancer wherein the disease is selected from multiple myeloma, breast cancer or prostate cancer.

The disclosure relates to a method of treating warm-blooded animals including mammals, especially humans, suffering from bone destruction associated with cancer by administering to a said animal in need of such treatment a dose effective against said disease of an HDAC inhibitor or a pharmaceutically acceptable salt thereof.

### Detailed Description of the Figures

Figure 1 illustrates LBH589 effects on tumor burden and body Weight in study #0879.
Figure 2 illustrates LBH589 effects on tumor burden and body Weight in study #0942.
Figure 3 illustrates LBH589 effects on time to clinical endpoint in #0942.
Figure 4 illustrates MicroCT scanning and trabecular bone measurement region of interest.
Figure 5 describes LBH589 effects on tibial trabecular bone in Study #879 and #0942.
Figure 6 describes LBH589 effects on tibial cortical bone.
Figure 7 describes LBH589 effects on serum bio-marker TRACP5b (0879).

### Detailed Description of the Invention

The HDAC inhibitor compound for use in the invention is 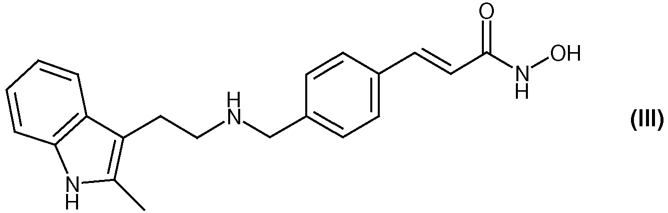 , i.e. the compound which is *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof.

The compound described above is often used in the form of a pharmaceutically acceptable salt. Pharmaceutically acceptable salts include, when appropriate, pharmaceutically acceptable base addition salts and acid addition salts, e.g., metal salts, such as alkali and alkaline earth metal salts, ammonium salts, organic amine addition salts and amino acid addition salts and sulfonate salts. Acid addition salts include inorganic acid addition salts, such as hydrochloride, sulfate and phosphate; and organic acid addition salts, such as alkyl sulfonate, arylsulfonate, acetate, maleate, fumarate, tartrate, citrate and lactate. Examples of metal salts are alkali metal salts, such as lithium salt, sodium salt and potassium salt; alkaline earth metal salts, such as magnesium salt and calcium salt, aluminum salt and zinc salt. Examples of ammonium salts are ammonium salt and tetramethylammonium salt. Examples of organic amine addition salts are salts with morpholine and piperidine. Examples of amino acid addition salts are salts with glycine, phenylalanine, glutamic acid and lysine. Sulfonate salts include mesylate, tosylate and benzene sulfonic acid salts.

This HDAC inhibitor and its synthesis are disclosed in WO 02/22577.

An HDAC inhibitor as used in the present invention displays in the assay described above preferably an IC₅₀ value between 50 and 2500 nM, more preferably between 250 and 2000 nM, and most preferably between 500 and 1250 nM.

The term "treatment", as used herein, comprises the treatment of patients having bone destruction caused by cancer, inflammatory diseases and osteoporosis.

In one embodiment, *N*-hydroxy-3-[4-[[[2-(2-methyl-1*H*-indol-3-yl)-ethyl]-amino]methyl]phenyl]-2*E*-2-propenamide or a pharmaceutically acceptable salt thereof is used to treat bone destruction associated with multiple myeloma. Hence, the invention relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof for the preparation of a medicament for the treatment of bone destruction associated with multiple myeloma. The invention also relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof in the treatment of bone destruction associated with multiple myeloma. The disclosure relates to a method of treating warm-blooded animals including mammals, especially humans, suffering from bone destruction associated with multiple myeloma by administering to a said animal in need of such treatment a dose effective against said disease of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof or a pharmaceutically acceptable salt thereof.

In another embodiment, *N*-hydroxy-3-[4-[[[2-(2-methyl-1*H*-indol-3-yl)-ethyl]-amino]methyl]phenyl]-2*E*-2-propenamide or a pharmaceutically acceptable salt thereof is used to treat bone destruction associated with breast cancer. Hence, the invention relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof for the preparation of a medicament for the treatment of bone destruction associated with breast cancer. The invention also relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof in the treatment of bone destruction associated with breast cancer. The disclosure relates to a method of treating warm-blooded animals including mammals, especially humans, suffering from bone destruction associated with breast cancer by administering to a said animal in need of such treatment a dose effective against said disease of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof or a pharmaceutically acceptable salt thereof.

In another embodiment, *N*-hydroxy-3-[4-[[[2-(2-methyl-1*H*-indol-3-yl)-ethyl]-amino]methyl]phenyl]-2*E*-2-propenamide or a pharmaceutically acceptable salt thereof is used to treat bone destruction associated with prostate cancer. Hence, the invention relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof for the preparation of a medicament for the treatment of bone destruction associated with prostate cancer. The invention also relates to the use of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof in the treatment of bone destruction associated with prostate cancer. The disclosure relates to a method of treating warm-blooded animals including mammals, especially humans, suffering from bone destruction associated with prostate cancer by administering to a said animal in need of such treatment a dose effective against said disease of *N*-hydroxy-3-[4-[[[2-(2-methyl-1*H*-indol-3-yl)-ethyl]-amino]methyl]phenyl]-2*E*-2-propenamide or a pharmaceutically acceptable salt thereof.

The person skilled in the pertinent art is fully enabled to select relevant test models to prove the beneficial effects mentioned herein. The pharmacological activity of such a compound may, e.g., be demonstrated by means of the Examples described below, by *in vitro* tests and *in vivo* tests or in suitable clinical studies. The efficacy of the treatment is determined in these studies, e.g., by evaluation of the tumor sizes every 4 weeks, with the control achieved on placebo.

The effective dosage of the HDAC inhibitor may vary depending on the particular compound or pharmaceutical composition employed, on the mode of administration, the type of the disease being treated or its severity. The dosage regimen is selected in accordance with a variety of further factors including the renal and hepatic function of the patient. A physician, clinician or veterinarian of ordinary skill can readily determine and prescribe the effective amount of compounds required to prevent, counter or arrest the progress of the condition.

### Example 1

Female SCID-beige mice were injected with MM1S cells (2 × 10⁶) intravenously (iv) into the tail vein on Day 0. Treatment was initiated on Day 10 when the average tumor burden, as determined by bioluminescence, reached approximately 8.0 x10⁵ - 1.0 x10⁶ photons per second. All treatment groups consisted of 8 animals. LBH589 was dosed at 15 mg/kg ip qdX5 for 3 weeks in the first study. In the second study, LBH589 was dosed at 10 mg/kg ip qdX5 for 6 weeks and 20 mg/kg ip qdX5 for 5 weeks. Tumor burden and body weights were recorded once a week during the active dosing period. *In vivo* micro-computed tomography (microCT) images were acquired in live animals on day 32 and 33 (Study #1) or Day 34 and 35 (Study #2). In the second study, animals were individually monitored until achievement of clinical endpoint.

### In vivo microCT or uCT analysis

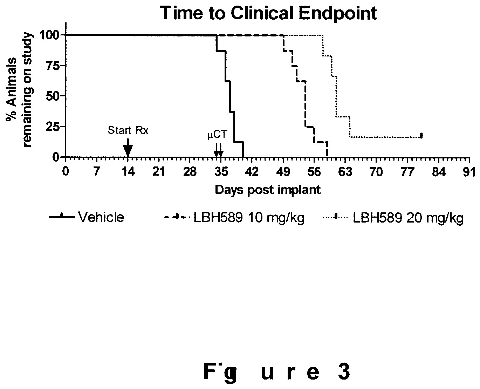

Animals were anesthetized with 2% isoflurane mixed with oxygen (2 L/min.) and then placed in a mouse holder (custom made, Peter Ingold, NIBR Basel) specifically designed to align both tibiae and mounted into an *in vivo* high resolution microCT scanner (VivaCT40, Scanco, Switzerland). To insure correct positioning of the mouse, a scout view of bilateral tibia bones and knee joints was taken and the region of interest (ROI, 2.23 mm in length) was positioned to start at the growth plate extending distally over the area of the trabecular bone (Figure 3). The scanner was set to a nominal isotropic voxel size of 21 µm, referred to as *medium*/*standard resolution.* The X-ray tube was operated at 55 kVp and 145 mA with a focal spot size of 5 µm. Five hundred projection images were acquired per scan with an integration time of 180 ms. Tomographic images were reconstructed on a VMS cluster (HP Alpha, HP, Palo Alto, USA) in 1024 x 1024 pixel matrices using a conebeam back projection procedure resulting in 315 axial slices.

For determination of trabecular and cortical bone features, a 2.23 mm region of interest was placed to start at the growth plate extending distally. 106 axial slices were obtained using a µ-CT VivaCT40 Scanner (SCANCO, Switzerland) with 55kv, 145 mA, 180 ms integration time and 21 µm resolution. Trabecular bone density (BV/TV) was measured in a 0.735 mm region of a tibia (9 slices proximal and 25 slices distal from the tibial tuberosity) using SCANCO software (SCANCO, Switzerland) with a threshold of 275 is used to define calcified bone volume (BV). Cortical bone density (BV/TV) was measured in a 1.5 mm region of a tibia (15 slices proximal and 55 slices distal from the tibial tuberosity) using SCANCO software (SCANCO Switzerland) with a threshold of 275. Three-dimensional analysis was performed on the determined regions utilizing the SCANCO operational software. All treatment groups were scanned over the course of two days, with equal numbers of animals from each treatment group scanned each day.

### Serum bio-marker analysis

A serum marker of bone metabolism, TRACP5B, was assessed for mouse serum changes. The MouseTRAP™ Assay kit is an ELISA assay (Cat#SB-TR103, IDS Fountain Hills, AZ). Briefly, polyclonal mouse TRACP5B antibodies are incubated in 96 well plates coated with anti-rabbit IgG. This ELISA kit is specific for mouse TRACP5B only. This assay has a reported sensitivity of 0.1 U/L.

### LBH589 effects on tumor burden

Following tail vein injection, MM1S cells proliferated and tumor burden increased over 1,400 to 2,300-fold as determined by bioluminescent readout. MM1S cells localized to bone resulting in multifocal bone lesions in the vertebrae, ribs, skull, pelvis and long bones consistent with human clinical presentation.

The mean relative change in tumor burden expressed as luciferase flux (photons per second) are shown in Tables 1 and 2:

**Table 1. Response Summary for Study #0879 31 Days After Implantation**

| **DRUGS** | | | **TUMORS** | | | | | **ANIMALS** | |
|---|---|---|---|---|---|---|---|---|---|
| Compound | Regimen & Route | Dose (mg/kg) | Delta Mean Tumor Burden (photons/sec) (mean ± SEM) | | | % T/C | % Regression | Delta % Body Wt. | Dead/ Total |
| Vehicle | D5W, IP, qdx5 | N/A | 1.3E + 9 | ± | 261.8E + 6 | NE | NE | -3.8 ± 1.47 | 0/8 |
| LBH589 | IP, qdx5 | 15 | 292.4E+6 | ± | 92.6E+6 | 22 | None | -10.5 ± 0.99 | 1/8 |

Treatments were started on Day 10 post-iv tail implantation (2.0 million cells/animal). LBH589 was administered ip, at 15 mg/kg, 5 times per week for 3 weeks. Vehicle control (D5W) was administered ip times per week, for 3 weeks. Initial group size: 8 animals. Final efficacy data and body weight change were calculated 72 hours post-last dose.

**Table 2. Response Summary for Study #0942 35 Days After Implantation**

| **DRUGS** | | | **TUMORS** | | | | | **ANIMALS** | |
|---|---|---|---|---|---|---|---|---|---|
| Compound | Regimen & Route | Dose (mg/kg) | Delta Mean Tumor Burden (photons/sec) (mean ± SEM) | | | % T/C | % Regression | Delta % Body Wt. | Dead / Total |
| Vehicle | D5W, IP, qdx5 | N/A | 1.1E+9 | ± | 171.4E+6 | NE | NE | 2 ± 1.81 | 1/8 |
| LBH589 | IP, qdx5 | 10 | 348.5E+6 | ± | 49.6E+6 | 31 | None | -11.3 ± 0.89 | 0/8 |
| LBH589 | IP, qdx5 | 20 | 107.7E+6 | ± | 42.6E+6 | 9 | None | -13.1 ± 1.22 | 0/8 |

Treatments were started on Day 10 post-iv tail implantation (2.0 million cells/animal). LBH589 was administered ip, at 10 or 20 mg/kg, 5 times per week for 4 weeks. Vehicle control (D5W) was administered ip times per week, for 3 weeks. Initial group size: 8 animals.

Statistical analyses of final tumor burden are presented in Tables 3 and Table 4.

**Table 3. 0879 Statistics of Day 31 Delta Tumor Burden**

| **Treatments (#0879)** | **Vehicle** | **LBH589 (15)** |
|---|---|---|
| Vehicle | X | S |
| LBH589 (15) | | x |

| | | |
|---|---|---|
| Students T-Test S = P<0.01 | | |

**Table 4. 0942 Statistics of Day 35 Delta Tumor Burden**

| **Treatments (#0879)** | **Vehicle** | **LBH589 (10)** | **LBH589 (20)** |
|---|---|---|---|
| Vehicle | X | S | S |
| LBH589 (10) | | X | NS |
| LBH589 (20) | | | X |

| | | | |
|---|---|---|---|
| ANOVA + Dunnett's Method Post-hoc test S = P<0.05 NS = Not Significant | | | |

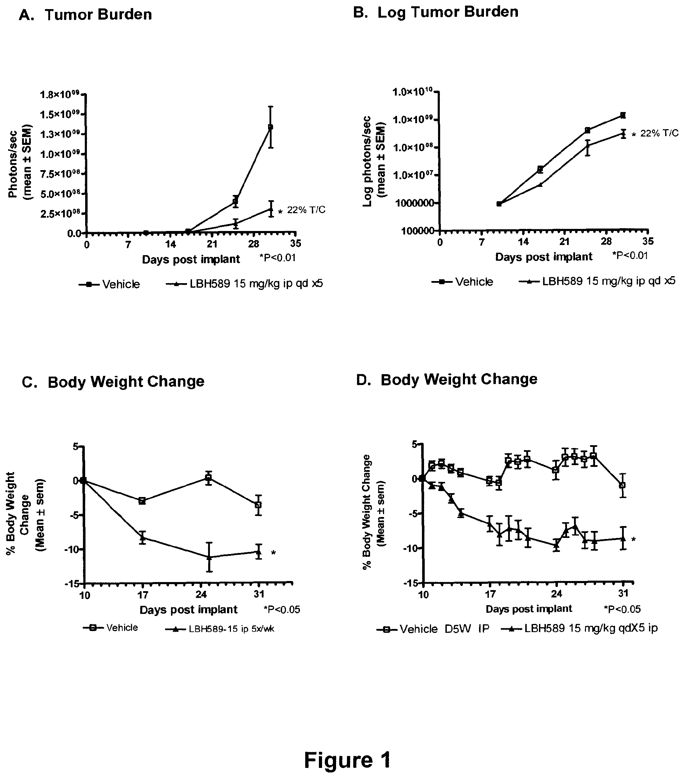

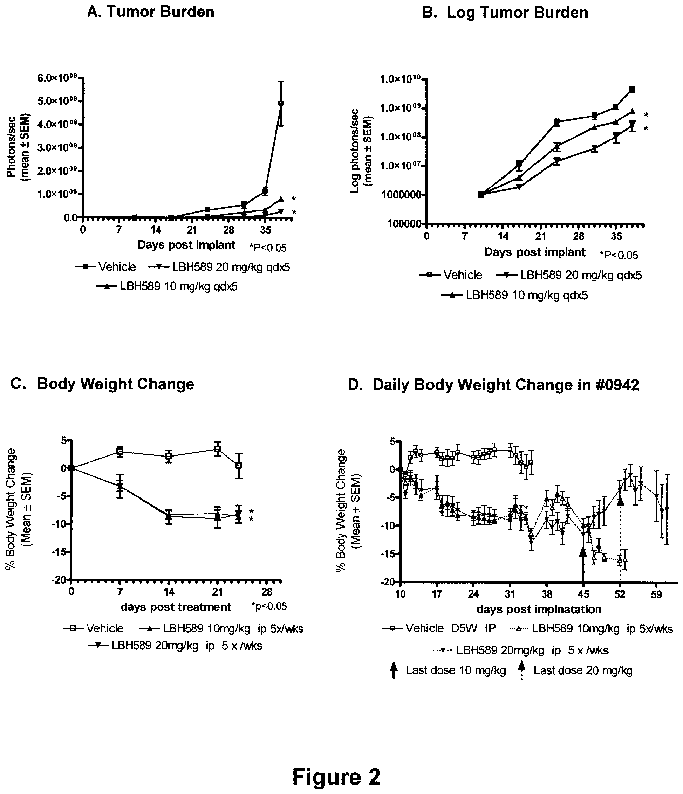

As illustrated in Figure 1, Figure 2, Table 1 and Table 2, tumor burden increased greater than 1,400-fold over the 4-5 week post-implantation period in the D5W-treated controls groups in Study #0879 and over 2,300-fold over the 5-week post-implantation period in the D5W-treated control groups in Study #0942.

With respect to Figure 2, treatments were started on Day 11 post-iv tail implantation (2.0 million cells/animal). NVP-LBH589-CU was administered ip, at 15 mg/kg (A), or 10 and 20 mg/kg (B), 5 times a week (qd x 5/wk) for 4 weeks. Vehicle control (D5W) was administered ip 5 times a week (qd x 5/wk), for 4 weeks. Bortezomib was administered iv, at 0.2 mg/kg, once per week (qwk) or 1 mg/kg, twice per week in Study #0879 for 4 weeks (biwk) (B). Initial group size: 8 animals. Final efficacy data are shown in the A panel for Study #0879 and B panel for Study #0942. Body weight changes were calculated on 24 hours post-last dose for each study (right panels).

LBH589 treatment at 15 mg/kg qdX5 alone resulted in a reduction in tumor burden by ∼78% on Day 31 in Study #0879. LBH589 treatment at 10 mg/kg qdX5 or 20 mg/kg qdX5 alone resulted in a reduction in tumor burden by ∼79% and ∼91%, respectively on Day 35 in Study #0942. The reduction in tumor burden by LBH589 was statistically significant in both studies.

### LBH589 effects on time to endpoint

The ability of LBH589 to extend the time to clinical endpoint was evaluated in Study #0942. Each animal was monitored daily for progression of signs of disease progression, including mobility and general health. Animals were scored on a clinical scale from 0-4. Endpoint was achieved when animals achieved a clinical score of 3. The effects of LBH589 on increasing time to endpoint are shown in Figure 3 and Table 5:

**Table 5. Effect of LBH589 on Median Time to Clinical Endpoint**

| **Treatment** | **N failed** | **N Censored** | **Median (days)** | **95%CI** |
|---|---|---|---|---|
| Vehicle | 8 | 0 | 37 | (34, 38) |
| LBH589 10 mg/kg | 8 | 0 | 54 | (49, 56) |
| LBH589 20 mg/kg | 5* | 1** | 61 | (58, -) |

| | | | | |
|---|---|---|---|---|
| *2 animals were found dead on Day 45 after implantation. Animals exhibited 15% body weight loss and abdominal distension, but no clinical symptoms of bone disease prior to death. Deaths were ruled as treatment related and removed from analysis. **One animal exhibited no signs of disease by 80 days post implantation and was censored | | | | |

Two of the eight animals treated with LBH589 at 20 mg/kg that died on Day 45 did not demonstrate signs of bone disease prior to death and were ruled as treatment related deaths. These animals were removed from analysis due to treatment related deaths. One of the remaining six animals did not exhibit any symptoms of disease 80 days after implant, when the observations were terminated and was censored in endpoint analysis. The median time to endpoint for the vehicle treated animals was 37 days. LBH589 dosed at 10 and 20 mg/kg resulted in median time to clinical endpoint of 54 and 61 days, respectively. The dose response increase in median time to achieve endpoint was significantly different, as evidenced by the non-overlapping 95% confidence intervals.

### LBH589 effects on trabecular bone

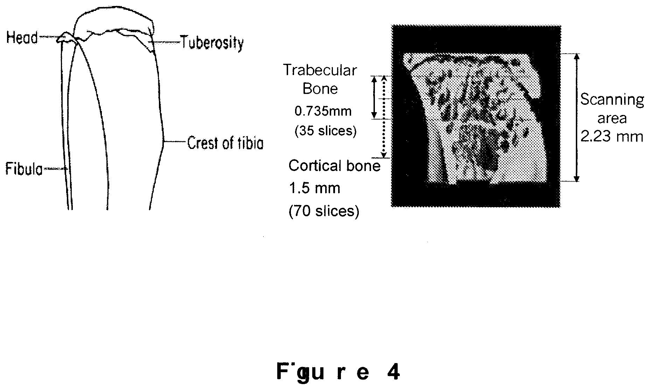

MicroCT was used to evaluate the effects on trabecular bone of LBH589 in MM1S tumor bearing mice. The regions of interest and representative images are shown in Figure 4.

Figure 4 describes a 2.3 mm region of interest was placed to start at the growth plate extending distally. 106 axial slices were obtained using a µ-CT VivaCT40 Scanner (SCANCO, Switzerland) with 55 kv, 145 mA, 180 ms integration time and 21 µm resolution. Trabecular bone density, bone volume/total volume (BV/TV), was measured in a 0.735 mm region of a tibia (10 slices proximal and 25 slices distal from the tibial tuberosity) using SCANCO software (SCANCO, Switzerland) with a threshold of 275. Cortical bone density, bone volume/total volume (BV/TV), was measured in a 1.5 mm region of a tibia (15 slices proximal and 55 slices distal from the tibial tuberosity) using SCANCO software (SCANCO Switzerland) with a threshold of 275.

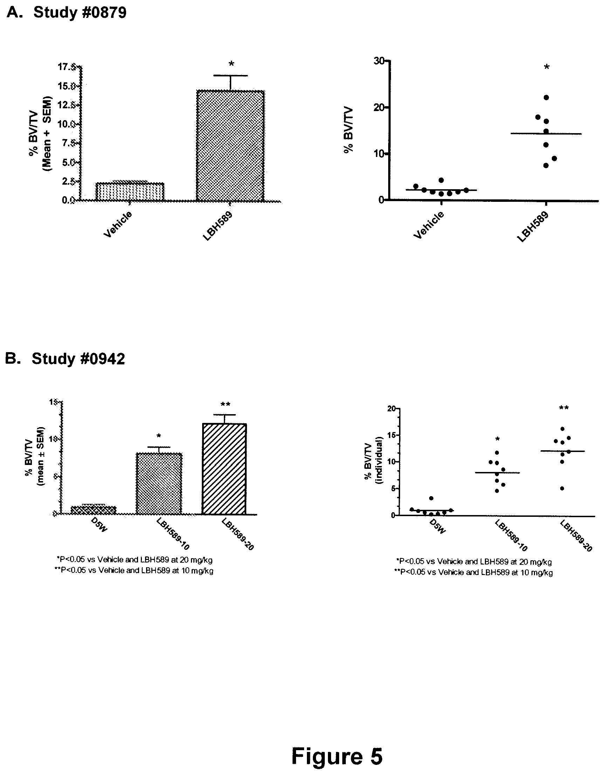

The mean trabecular bone density (BV/TV) and percent change (treated as a percent of control) are shown in Figure 5 and Tables 6 and 7.

**Table 6. 0879 Delta Mean Trabecular Density**

| **Treatments** | **Vehicle** | **LBH589 (15 mg/kg)** |
|---|---|---|
| Number of Values | 8 | 7 |
| Mean | 2.23 | 14.48 |
| Standard Error | 0.344 | 1.97 |
| % Relative mean change treated/vehicle | n/a | 549% |

**Table 7. 0942 Delta Median Trabecular Density**

| **Treatments** | **Vehicle** | **LBH589 (10 mg/kg)** | **LBH589 (20 mg/kg)** |
|---|---|---|---|
| Number of Values | 8 | 8 | 8 |
| Median | 0.64 | 8.23 | 12.89 |
| % Relative median change treated/vehicle | n/a | 1186% | 1914% |

### Statistical analysis of trabecular bone density are presented in Tables 8 and 9.

**Table 8. 0879 Statistics of Trabecular Bone Density**

| **Treatments** | **Vehicle** | **LBH589 (15 mg/kg)** |
|---|---|---|
| Vehicle | X | S |
| LBH589 (15) | | X |

| | | |
|---|---|---|
| Students T-Test S = P<0.05 | | |

**Table 9. 0942 Statistics of Trabecular Bone Density**

| **Treatments (#0879)** | **Vehicle** | **LBH589 (10 mg/kg)** | **LBH589 (20 mg/kg)** |
|---|---|---|---|
| Vehicle | X | S | S |
| LBH589 (10) | | X | S |
| LBH589 (20) | | | X |

| | | | |
|---|---|---|---|
| Wilcoxon / Kruskal-Wallis Test with Tukey-Kramer Post-hoc multiple comparison S = P<0.05 | | | |

LBH589 at 15 mg/kg resulted in a statistically significant 5.5-fold increase in mean trabecular bone density after 3 weeks of treatment. In Study #0942, LBH589 dosed at 10 and 20 mg/kg resulted in a statistically significant increase in median trabecular bone density of 11.8- and 19.1-fold, respectively, after 4 weeks of treatment.

Figure 5 describes that trabecular bone density (%BV/TV) was analyzed. In panel A and B, the bar graphs represent the mean average ± SEM of the tibial trabecular bone density (%BV/TV) for Study #0879 and #0942. The right panels are the results of individual animals are represented in the graphs above. * indicates statistical significance from vehicle control at the same time point (p < 0.05).

### LBH589 effects on cortical bone

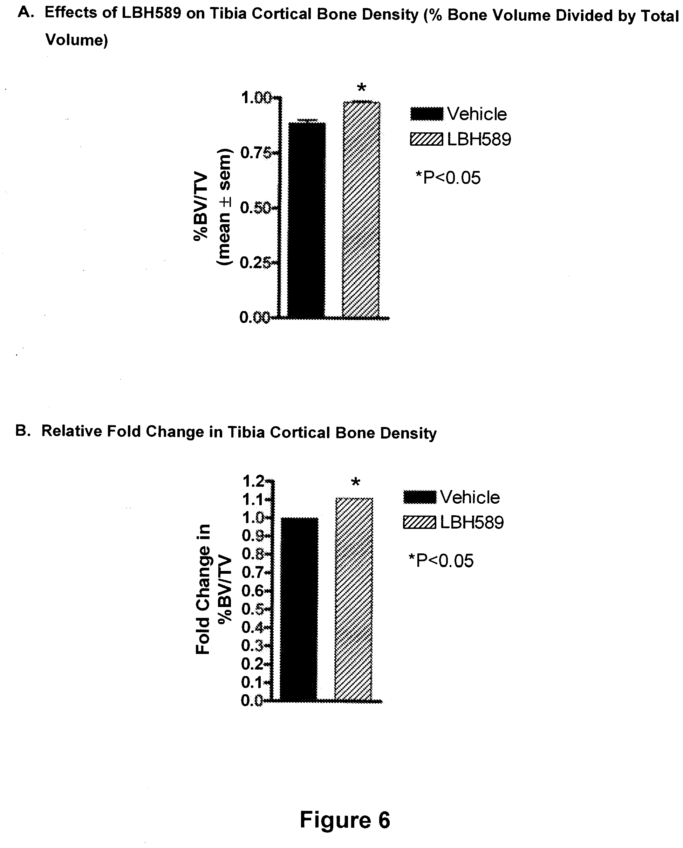

The effects on cortical bone of LBH589 as a single agent was evaluated by microCT analysis in Study #0879. Quantitative analysis of the cortical bone density and their relative differences for Study #0879 are represented in Figure 6 and Table 10.

**Table 10. 0879 Delta Mean Cortical Density**

| **Treatments** | **Vehicle** | **LBH589 (15 mg/kg)** |
|---|---|---|
| Number of values | 8 | 7 |
| Mean | 88.7% | 98.3% |
| Standard error | 1.4% | 0.4% |
| % Relative mean change treated/vehicle | N/A | 10.8% |

Statistical analysis is presented in Table 11. Treatment with LBH589 resulted in statistically significant 10.8% increase in cortical bone density relative to vehicle treated animals.

**Table 11. 0879 Statistics of Cortical Bone Density**

| **Treatments** | **Vehicle** | **LBH589 (15 mg/kg)** |
|---|---|---|
| Vehicle | X | S |
| LBH589 (15 mg/kg) | | X |

| | | |
|---|---|---|
| Students T-Test S = P<0.0001 | | |

### Serum biomarker evaluation

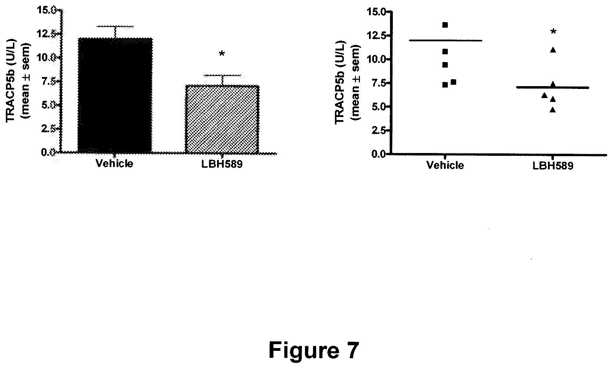

TRACP5B serum levels were evaluated as a measure of osteoclast activity. The level of a TRACP5B was analyzed in Figure 7 and Table 12.

**Table 12. 0879 Delta Mean \ TRACP5B Serum Levels**

| **Treatments** | **Vehicle** | **LBH589 (15 mg/kg)** |
|---|---|---|
| Number of Values | 8 | 5 |
| Mean | 12.0 | 7.1 |
| Standard Error | 1.3 | 1.1 |
| % Relative mean change treated/vehicle | n/a | -41% |

Serum bio-marker TRACP5b was analyzed as described in the Methods. In the left panel, the bar graph represents the mean average ± SEM. * indicates statistical significance from controls (p < 0.05).

Serum levels of TRACP5B in this study were significantly decreased by 41% in animals treated with LBH589 alone as compared to vehicle treated animals, Table 13:

**Table 13 0879 Statistics of TRACP5B Serum Levels**

| **Treatments** | **Vehicle** | **LBH589 (15 mg/kg)** |
|---|---|---|
| Vehicle | X | S |
| LBH589 (15) | | X |

| | | |
|---|---|---|
| Students T-Test S = P<0.05 | | |

## Claims

1. Use of a histone deacetylase (HDAC) inhibitor for the preparation of a medicament for the treatment and/or prevention of bone destruction caused by a proliferative disease, wherein the proliferative disease is selected from multiple myeloma, breast cancer or prostate cancer, wherein the HDAC inhibitor is *N*-hydroxy-3-[4-[[[2-(2-methyl-1*H*-indol-3-yl)-ethyl]-amino]methyl]phenyl]-2*E*-2-propenamide having the formula (III): 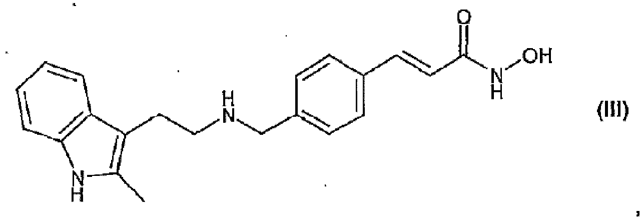 or a pharmaceutically acceptable salt thereof.

2. The use according to claim 1 for the preparation of a medicament for the treatment of bone destruction caused by a proliferative disease.

3. The use according to claim 1 for the preparation of a medicament for the prevention of bone destruction caused by a proliferative disease.

4. The use according to any one of claims 1-3, wherein the proliferative disease is multiple myeloma.

5. The use according to any one of claims 1-3, wherein the proliferative disease is breast cancer.

6. The use according to any one of claims 1-3, wherein the proliferative disease is prostate cancer.

7. A histone deacetylase (HDAC) inhibitor for use in the treatment and/or prevention of bone destruction caused by a proliferative disease, wherein the proliferative disease is selected from multiple myeloma, breast cancer or prostate cancer, wherein the HDAC inhibitor is *N*-hydroxy-3-[4-[[[2-(2-methyl-1*H*-indol-3-yl)-ethyl]-amino]methyl]phenyl]-2*E*-2-propenamide having the formula (III): 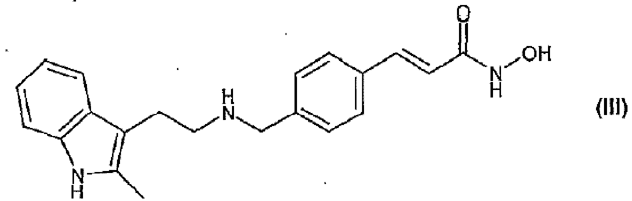 or a pharmaceutically acceptable salt thereof.

8. The HDAC inhibitor for use according to claim 7, wherein the use is use in the treatment of bone destruction caused by a proliferative disease.

9. The HDAC inhibitor for use according to claim 7, wherein the use is use in the prevention of bone destruction caused by a proliferative disease.

10. The HDAC inhibitor for use according to any one of claims 7-9, wherein the proliferative disease is multiple myeloma.

11. The HDAC inhibitor for use according to any one of claims 7-9, wherein the proliferative disease is breast cancer.

12. The HDAC inhibitor for use according to any one of claims 7-9, wherein the proliferative disease is prostate cancer.

13. The histone deacetylase (HDAC) inhibitor which is *N*-hydroxy-3-[4-[[[2-(2-methyl-1*H*-indol-3-yl)-ethyl]-amino]methyl]phenyl]-2*E*-2-propenamide having the formula (III): 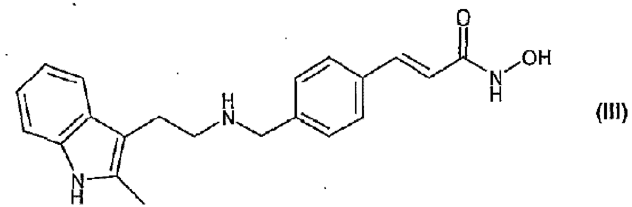 or a pharmaceutically acceptable salt thereof, for use in the treatment of bone destruction caused by multiple myeloma.

## Patentansprüche

1. Verwendung eines Histondeacetylase(HDAC)-Inhibitors zur Herstellung eines Medikaments zur Behandlung und/oder Prävention von durch eine proliferative Krankheit verursachter Knochenzerstörung, wobei die proliferative Krankheit aus multiplem Myelom, Brustkrebs und Prostatakrebs ausgewählt ist, wobei es sich bei dem HDAC-Inhibitor um *N*-Hydroxy-3-[4-[[[2-(2-methyl-1*H*-indol-3-yl)ethyl]amino]methyl]phenyl]-2*E*-2-propenamid mit der Formel (III): 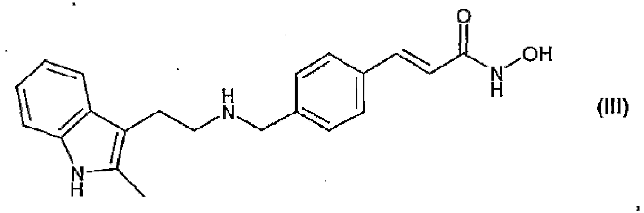 oder ein pharmazeutisch unbedenkliches Salz davon handelt.

2. Verwendung nach Anspruch 1 zur Herstellung eines Medikaments zur Behandlung von durch eine proliferative Krankheit verursachter Knochenzerstörung.

3. Verwendung nach Anspruch 1 zur Herstellung eines Medikaments zur Prävention von durch eine proliferative Krankheit verursachter Knochenzerstörung.

4. Verwendung nach einem der Ansprüche 1-3, wobei es sich bei der proliferativen Krankheit um multiples Myelom handelt.

5. Verwendung nach einem der Ansprüche 1-3, wobei es sich bei der proliferativen Krankheit um Brustkrebs handelt.

6. Verwendung nach einem der Ansprüche 1-3, wobei es sich bei der proliferativen Krankheit um Prostatakrebs handelt.

7. Histondeacetylase(HDAC)-Inhibitor zur Verwendung bei der Behandlung und/oder Prävention von durch eine proliferative Krankheit verursachter Knochenzerstörung, wobei die proliferative Krankheit aus multiplem Myelom, Brustkrebs und Prostatakrebs ausgewählt ist, wobei es sich bei dem HDAC-Inhibitor um *N*-Hydroxy-3-[4-[[[2-(2-methyl-1*H*-indol-3-yl)ethyl]amino]methyl]phenyl]-2*E*-2-propenamid mit der Formel (III): 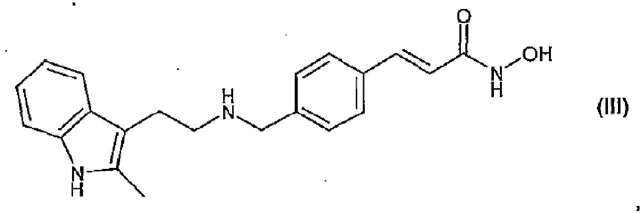 oder ein pharmazeutisch unbedenkliches Salz davon handelt.

8. HDAC-Inhibitor zur Verwendung nach Anspruch 7, wobei es sich bei der Verwendung um die Verwendung bei der Behandlung von durch eine proliferative Krankheit verursachter Knochenzerstörung handelt.

9. HDAC-Inhibitor zur Verwendung nach Anspruch 7, wobei es sich bei der Verwendung um die Verwendung bei der Prävention von durch eine proliferative Krankheit verursachter Knochenzerstörung handelt.

10. HDAC-Inhibitor zur Verwendung nach einem der Ansprüche 7-9, wobei es sich bei der proliferativen Krankheit um multiples Myelom handelt.

11. HDAC-Inhibitor zur Verwendung nach einem der Ansprüche 7-9, wobei es sich bei der proliferativen Krankheit um Brustkrebs handelt.

12. HDAC-Inhibitor zur Verwendung nach einem der Ansprüche 7-9, wobei es sich bei der proliferativen Krankheit um Prostatakrebs handelt.

13. Histondeacetylase(HDAC)-Inhibitor, bei dem es sich um *N*-Hydroxy-3-[4-[[[2-(2-methyl-1*H*-indol-3-yl)ethyl]amino]methyl]phenyl]-2*E*-2-propenamid mit der Formel (III) : 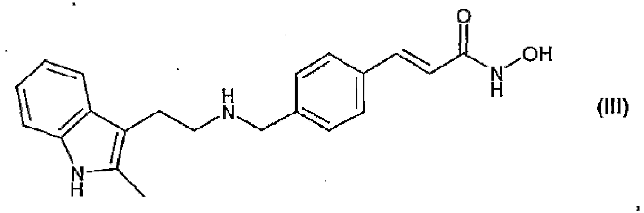 oder ein pharmazeutisch unbedenkliches Salz davon handelt, zur Verwendung bei der Behandlung von durch multiples Myelom verursachter Knochenzerstörung.

## Revendications

1. Utilisation d'un inhibiteur d'histone désacétylase (HDAC) dans l'élaboration d'un médicament destiné au traitement prophylactique et/ou thérapeutique de la destruction osseuse provoquée par une maladie proliférative, où la maladie proliférative est choisie parmi le myélome multiple, le cancer du sein et le cancer de la prostate, où l'inhibiteur de HDAC est le *N*-hydroxy-3-[4-[[[2-(2-méthyl-1*H*-indol-3-yl)-éthyl]-amino]méthyl]phényl]-2*E*-2-propénamide répondant à la formule (III) : 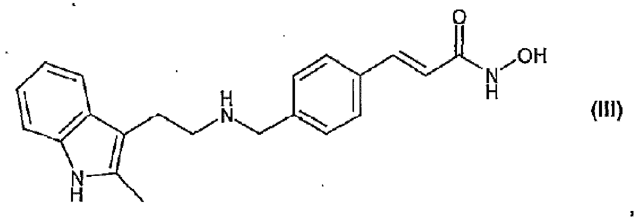 ou sel pharmaceutiquement acceptable de celui-ci.

2. Utilisation selon la revendication 1 dans l'élaboration d'un médicament destiné au traitement thérapeutique de la destruction osseuse provoquée par une maladie proliférative.

3. Utilisation selon la revendication 1 dans l'élaboration d'un médicament destiné au traitement prophylactique de la destruction osseuse provoquée par une maladie proliférative.

4. Utilisation selon l'une quelconque des revendications 1 à 3, où la maladie proliférative est le myélome multiple.

5. Utilisation selon l'une quelconque des revendications 1 à 3, où la maladie proliférative est le cancer du sein.

6. Utilisation selon l'une quelconque des revendications 1 à 3, où la maladie proliférative est le cancer de la prostate.

7. Inhibiteur d'histone désacétylase (HDAC) pour utilisation dans le traitement prophylactique et/ou thérapeutique de la destruction osseuse provoquée par une maladie proliférative, où la maladie proliférative est choisie parmi le myélome multiple, le cancer du sein et le cancer de la prostate, où l'inhibiteur de HDAC est le *N*-hydroxy-3-[4-[[[2-(2-méthyl-1*H*-indol-3-yl)-éthyl]-amino]méthyl]phényl]-2*E*-2-propénamide répondant à la formule (III) : 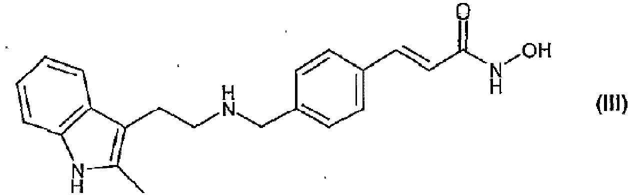 ou sel pharmaceutiquement acceptable de celui-ci.

8. Inhibiteur de HDAC pour utilisation selon la revendication 7, où l'utilisation est une utilisation dans le traitement thérapeutique de la destruction osseuse provoquée par une maladie proliférative.

9. Inhibiteur de HDAC pour utilisation selon la revendication 7, où l'utilisation est une utilisation dans le traitement prophylactique de la destruction osseuse provoquée par une maladie proliférative.

10. Inhibiteur de HDAC pour utilisation selon l'une quelconque des revendications 7 à 9, où la maladie proliférative est le myélome multiple.

11. Inhibiteur de HDAC pour utilisation selon l'une quelconque des revendications 7 à 9, où la maladie proliférative est le cancer du sein.

12. Inhibiteur de HDAC pour utilisation selon l'une quelconque des revendications 7 à 9, où la maladie proliférative est le cancer de la prostate.

13. Inhibiteur d'histone désacétylase (HDAC) qui est le *N*-hydroxy-3-[4-[[[2-(2-méthyl-1*H*-indol-3-yl)-éthyl]-amino]méthyl]phényl]-2*E*-2-propénamide répondant à la formule (III) : 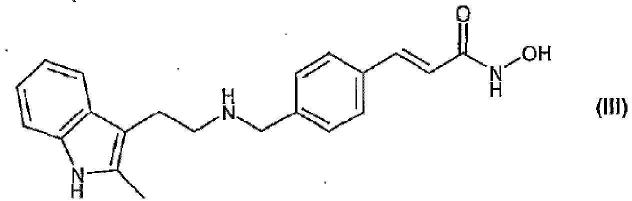 ou sel pharmaceutiquement acceptable de celui-ci, pour utilisation dans le traitement thérapeutique de la destruction osseuse provoquée par le myélome multiple.
